# EUROPÄISCHE PATENTSCHRIFT

(11) **EP 0 846 104 B1**
(45) Veröffentlichungstag und Bekanntmachung des Hinweises auf die Patenterteilung: **14.11.2001**
(21) Anmeldenummer: 96928408.2
(22) Anmeldetag: 05.08.1996
(51) Int. Cl.: C07C 327/22, A01N 37/36

(54) **ALKOXYACRYLSÄURETHIOLESTER ALS FUNGIZIDE**
ALKOXYACRYLIC ACID THIOL ESTERS USED AS FUNGICIDES
ESTERS DE THIOL D'ACIDE ALCOXYACRYLIQUE UTILISES COMME FONGICIDES

(30) Priorität: 17.08.1995 DE 19530199
(43) Veröffentlichungstag der Anmeldung: 10.06.1998
(73) Patentinhaber: BAYER AG, 51368 Leverkusen (DE)
(72) Erfinder: GAYER, Herbert, D-40789 Monheim (DE); GERDES, Peter, D-52080 Aachen (DE); HEINEMANN, Ulrich, D-42799 Leichlingen (DE); KRÜGER, Bernd-Wieland, D-51467 Bergisch Gladbach (DE); TIEMANN, Ralf, D-51375 Leverkusen (DE); STENZEL, Klaus, D-40595 Düsseldorf (DE); DUTZMANN, Stefan, D-40721 Hilden (DE); HÄNSSLER, Gerd, D-51381 Leverkusen (DE)
(86) Internationale Anmeldenummer: EP9603436
(87) Internationale Veröffentlichungsnummer: WO9707096

(56) Entgegenhaltungen:
- EP-A- 0 178 826
- EP-A- 0 226 917
- EP-A- 0 278 595
- EP-A- 0 432 503
- DE-A- 2 444 321
- DE-A- 2 840 589
- FR-A- 2 054 532
- GB-A- 2 051 043
- BIOCHEMICAL SOCIETY TRANSACTIONS, Bd. 21, Nr. 1, Februar 1993, LONDON, GB, Seite 1S XP000610171 T.E. WIGGINS: "The realtionship between structure and activity of the methoxyacrylate toxophore"
- JOURNAL OF ORGANIC CHEMISTRY, Bd. 56, Nr. 12, 7.Juni 1991, WASHINGTON, DC, US, Seiten 3955-3957, XP002018744 T. YAMATO, ET AL.: "Organic reactions catalysed by solid superacids. 5. Perfluorinated sulphonic acid resin (Nafion-H) catalysed intramolecular Friedel-Crafts acylation"
- JOURNAL OF HETEROCYCLIC CHEMISTRY, Bd. 29, Nr. 1, Januar 1992 - Februar 1992, PROVO, US, Seiten 11-16, XP002018745 D.H. KIM: "Synthesis of 5,6,8,9,14,14a- hexahydroisoquino[1,2-b][3]benzazpines"
- JOURNAL OF THE CHEMICAL SOCIETY, 1949, LETCHWORTH, GB, Seiten 1720-1724, XP002018746 G.A. SWAN: "The constitution of yohimbine and related alkaloids. Part III. The structure of sempervirine and some further observations on the structure of ketoyobyrine"
- JOURNAL OF ORGANIC CHEMISTRY, Bd. 26, Nr. 1, 24.Januar 1961, WASHINGTON, DC, US, Seiten 97-102, XP002018747 R.L. LETSINGER, ET AL.: "Reactions of 2-benzhydrylphenylacetic acid; a new pyrone synthesis"
- BULLETIN DE LA SOCIETE CHIMIQUE DE FRANCE, 1959, PARIS, FR, Seiten 638-643, XP002018748 J. RIGAUDY, ET AL.: "Cétones dérivées du dibenzo [a,d] cycloheptadiène. I. La dibenzo-2.3.-6,7 cycloheptadiènone-4,5"
- JOURNAL OF ORGANIC CHEMISTRY, Bd. 59, Nr. 14, 15.Juli 1994, WASHINGTON, DC, US, Seiten 3821-3829, XP002018749 W. KIRMSE, ET AL.: "Intramolecular reactivity of arylcarbenes: derivatives of o-tolylcarbene"
- TETRAHEDRON, Bd. 26, Nr. 2, Januar 1970, OXFORD, GB, Seiten 631-640, XP002018751 J.R. PRAHLAD, ET AL.: "Studies in sesquiterpenes - XLI. Isolongifolene (part2): dehydrogenation"
- LIEBIGS ANNALEN DER CHEMIE, Bd. 752, 1971, WEINHEIM, DE, Seiten 115-135, XP002018752 W. WALTER, ET AL.: "Über die Oxydationsprodukte von Thiocarbonsäureamiden, XXVII. Tautomerie zwischen Thioamid-S-oxiden und Sulfensäuren"
- JOURNAL OF MEDICINAL CHEMISTRY, Bd. 23, Nr. 5, Mai 1980, WASHINGTON, DC, US, Seiten 494-501, XP002018753 H.H. ONG, ET AL.: "Tricyclics with analgesic and antidepressant activity. 1. [[(Alkylamino)ethyl]thio]dibenz[b,f]- oxepins and 10,11-dihydro derivatives"
- BERICHTE DER DEUTSCHEN CHEMISCHEN GESELLSCHAFT, Bd. 56, 1923, WEINHEIM DE, Seiten 1242-2152, XP002018750 J. VON BRAUN, ET AL.: "Synthesen in der fett-aromatischen Reihe, XIV: Über das Homo-o-xylylenbromid"

## Beschreibung

Die Erfindung betrifft neue Alkoxyacrylsäurethiolester, mehrere Verfahren zu ihrer Herstellung und ihre Verwendung als Fungizide, sowie neue Zwischenprodukte und mehrere Verfahren zu deren Herstellung.

Es ist bereits bekannt geworden, daß bestimmte Alkoxyacrylsäurederivate, die den unten beschriebenen Alkoxyacrylsäurethiolestern konstitutionell ähnlich sind, fungizide Eigenschaften besitzen (vergleiche z. B. EP-A 226917 oder EP-A 370629 oder EP-A 398692). Die fungizide Wirkung dieser Verbindungen ist jedoch in vielen Fällen unbefriedigend.

Es wurden nun die neuen Alkoxyacrylsäurethiolester der allgemeinen Formel (I) gefunden, 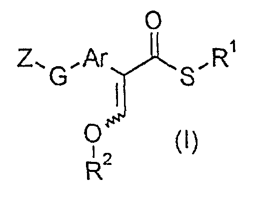 in welcher
- Ar: für ortho-Phenylen steht,
- G: für -O-CH₂- oder 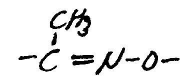 steht,
- R¹: für Methyl steht,
- R²: für Methyl steht, und
- Z: für jeweils gegebenenfalls einfach bis dreifach, gleich oder verschieden durch Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy, Methoximinomethyl, Ethoximinomethyl, substituiertes Phenyl, steht.

Die oben aufgeführten allgemeinen oder in den Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Weiterhin wurde gefunden, daß man die neuen Alkoxyacrylsäurethiolester der allgemeinen Formel (I) erhält, wenn man (Verfahren a)) Hydroxyacrylsäurethiolester der allgemeinen Formel (II) 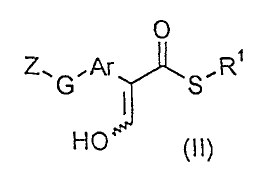 in welcher
- Ar, G, R¹ und Z: die oben angegebenen Bedeutungen haben,
mit einer Halogenverbindung der allgemeinen Formel (III)

X¹-R² (III)

in welcher
- X¹: für Halogen steht und
- R²: die oben angegebene Bedeutung hat,
gegebenenfalls in Gegenwart eines Säureakzeptors und gegebenenfalls in Gegenwart eines Verdünnungsmittels umsetzt.

Schließlich wurde gefunden, daß die neuen Alkoxyacrylsäurethiolester der allgemeinen Formel (I) eine sehr starke fungizide Wirkung zeigen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

Die erfindungsgemäßen Alkoxyacrylsäurethiolester sind durch die Formel (I) allgemein definiert.

Beispiele für die erfindungsgemäßen Verbindungen sind in den Tabellen 1 und 2 aufgeführt:

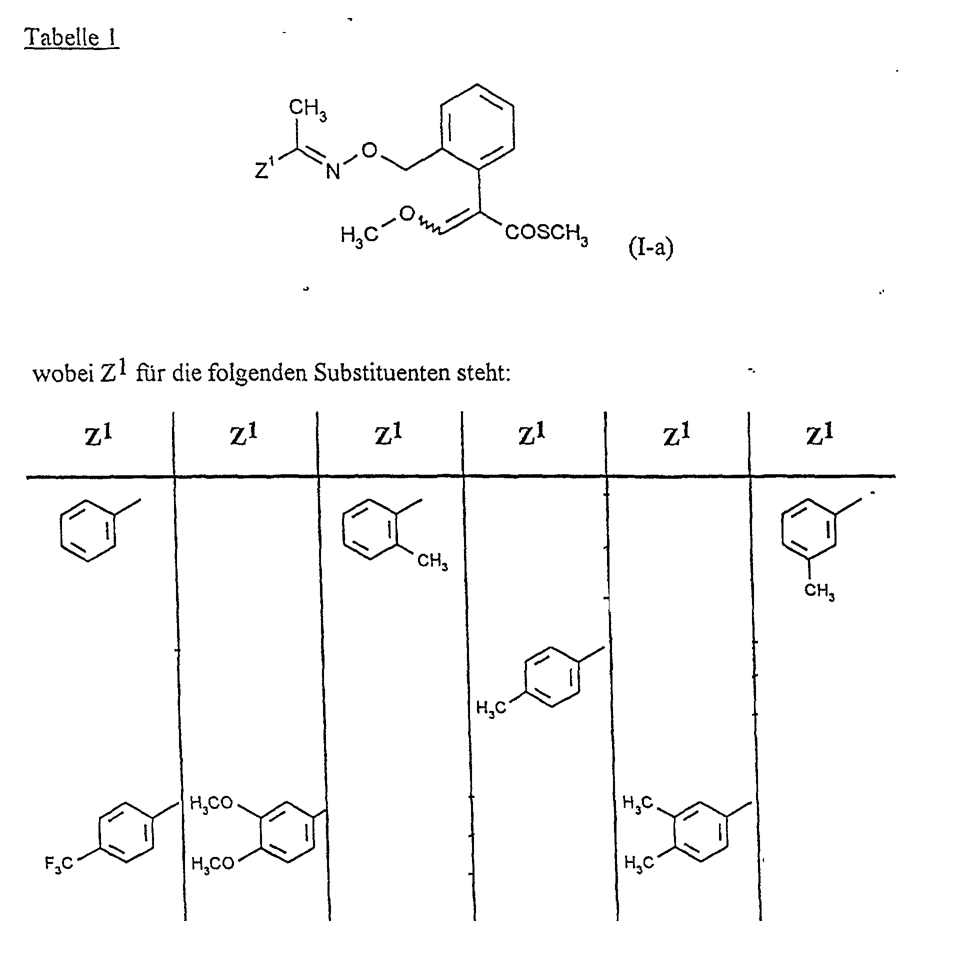

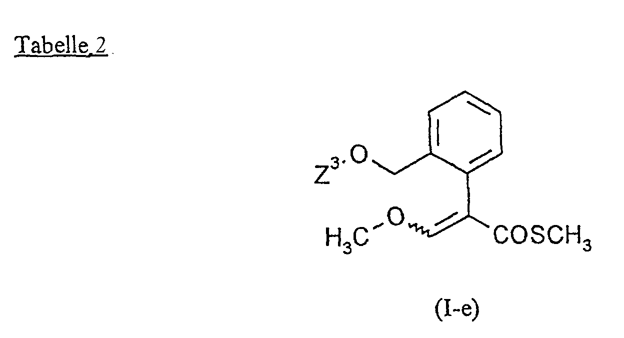

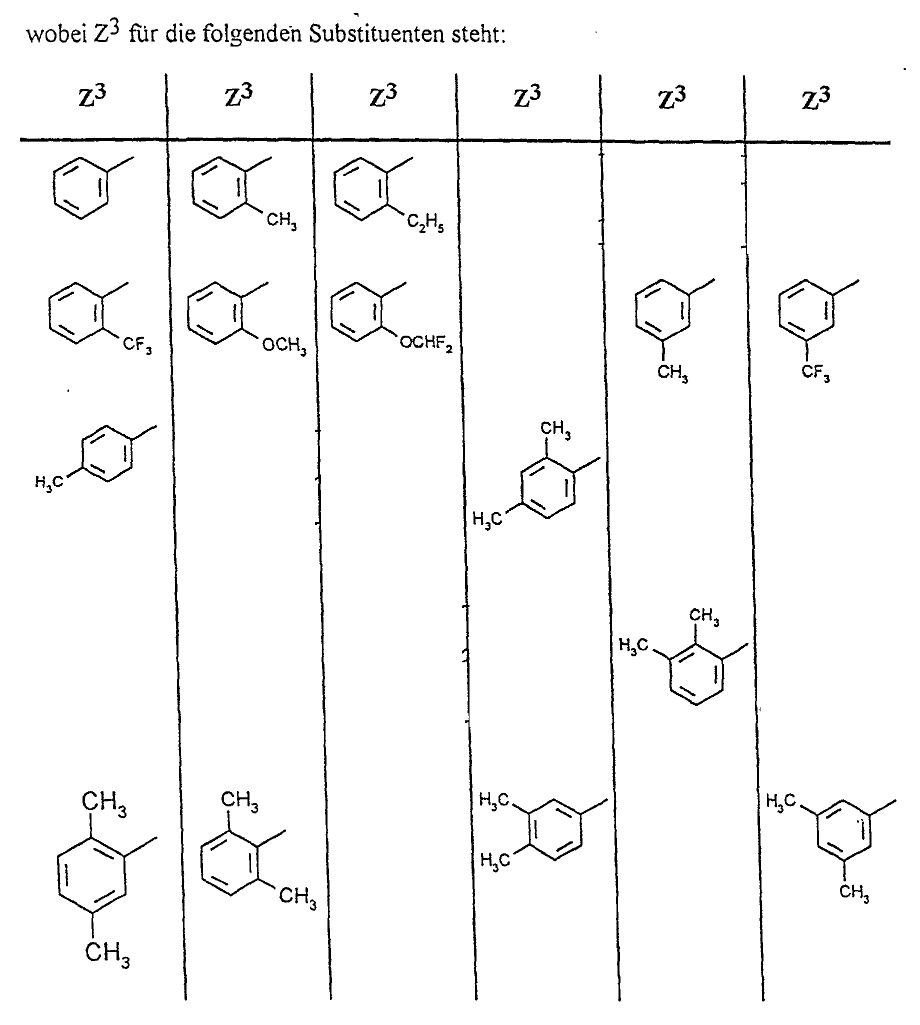

Die zur Durchführung des erfindungsgemäßen Verfahrens a) als Ausgangsstoffe benötigten Hydroxyacrylsäurethiolester sind durch die Formel (II) allgemein definiert. In dieser Formel (II) haben Ar, G, R¹ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Ar, G, R¹ und Z angegeben wurden.

Die Ausgangsstoffe der Formel (II) sind noch nicht bekannt, sie sind als neue Stoffe auch Gegenstand der vorliegenden Anmeldung.

Die Hydroxyacrylsäurethiolester der Formel (II) werden erhalten, wenn man (Verfahren b)) Essigsäurethiolester der allgemeinen Formel (IV), 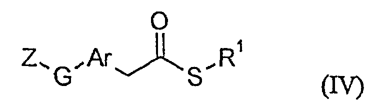 in welcher
- Ar, G, R¹ und Z: die oben angegebenen Bedeutungen haben,
mit einem Ameisensäurederivat, vorzugsweise Ameisensäuremethylester, Ameisensäureethylester, Orthoameisensäuretrimethylester, Orthoameisensäuretriethylester, Dimethylformamiddimethylacetal, Bis-(dimethylamino)-methoxymethan oder Bis-(dimethylamino)-t-butoxymethan, insbesondere Ameisensäuremethylester oder Bis-(dimethylamino)-t-butoxymethan, gegebenenfalls in Gegenwart einer Base, vorzugsweise eines Erdalkalimetall- oder Alkalimetallhydrids, -amids, -alkoholates, wie beispielsweise Natriumhydrid, Natriumamid, Natrium-methylat, Natrium-ethylat, Kalium-tert.-butylat und gegebenenfalls in Gegenwart eines Verdünnungsmittels, vorzugsweise eines Amids, wie N,N-Dimethylformamid oder eines Alkohols, wie Methanol, Ethanol, n- oder i-Propanol, n-, i-, sek- oder tert-Butanol, bei Temperaturen von 0 bis 150°C vorzugsweise von 10 bis 120°C, umsetzt.

Die zur Durchführung des erfindungsgemäßen Verfahrens b) als Ausgangsstoffe benötigten Essigsäurethiolester sind durch die Formel (IV) allgemein definiert. In dieser Formel (IV) haben Ar, G, R¹ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Ar, G, R¹ und Z angegeben wurden.

Die Essigsäurethiolester der Formel (IV) sind teilweise bekannt und können nach bekannten Methoden hergestellt werden (vergleiche z. B. EP-A 161529).

Neu und auch Gegenstand der vorliegenden Anmeldung sind die Essigsäurethiolester der Formel (IVa), 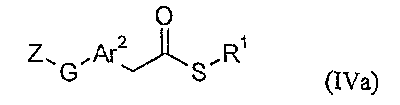 in welcher
- G, R¹ und Z: die oben angegebenen Bedeutungen haben und
- Ar²: für ortho-Phenylen, Pyridin-2,3-diyl oder Thiophen-2,3-diyl steht.

Die Essigsäurethiolester der Formel (IVa) werden erhalten, wenn man (Verfahren (c)) Säurehalogenide der allgemeinen Formel (V), 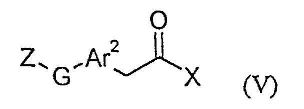 in welcher
- Ar², G und Z: die oben angegebenen Bedeutungen haben,
mit einem Thiol, bzw. einem Metallthiolat der Formel (VI),

R¹-S-M (VI)

in welcher
- R¹: die oben angegebene Bedeutung hat und
- M: für Wasserstoff oder ein Metalläquivalent steht,
gegebenenfalls in Gegenwart eines Säureakzeptors, vorzugsweise eines Erdalkalimetall- oder Alkalimetallhydrids, -hydroxids, -amids, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiäre Amins, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), und gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Nitrils, wie Acetonitril, Propionitril, n- oder i-Butyronitril oder Benzonitril; eines Amids, wie N,N-Dimethylformamid, N,N-Dimethylacetamid, N-Methylformanilid, N-Methylpyrrolidon oder Hexamethylphosphorsäuretriamid oder eines Sulfons, wie Sulfolan; bei Temperaturen von 0°C bis 150°C, vorzugsweise von 20°C bois 100°C, umsetzt.

Die neuen Essigsäurethiolester sind durch die Formel (IVa) allgemein definiert. In dieser Formel (IVa) haben G, R¹ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für G, R¹ und Z angegeben wurden. Ar² steht auch vorzugsweise und insbesondere bevorzugt für ortho-Phenylen, Pyridin-2,3-diyl oder Thiophen-2,3-diyl.

Die zur Durchführung des erfindungsgemäßen Verfahrens c) als Ausgangsstoffe benötigten Säurehalogenide sind durch die Formel (V) allgemein definiert. In dieser Formel (V) haben Ar², G und Z vorzugsweise bzw. insbesondere diejenigen Bedeutungen, die bereits im Zusammenhang mit der Beschreibung der erfindungsgemäßen Verbindungen der Formel (IVa) als bevorzugt bzw. als insbesondere bevorzugt für Ar², G und Z angegeben wurden.

Die Säurehalogenide der Formel (V) sind als neue Verbindungen ebenfalls Gegenstand der vorliegenden Anmeldung.

Die Säurehalogenide der Formel (V) werden erhalten (Verfahren d)), wenn man Säuren der Formel (VII) 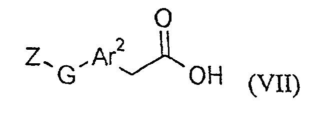 in welcher
Ar², G und Z die oben angegebenen Bedeutungen haben,
mit Halogenierungsmitteln, wie beispielsweise Thionylchlorid, Phosgen, Phosphorpentachlorid oder Phosphoroxychlorid, gegebenenfalls in Gegenwart eines Verdünnungsmittels, wie beispielsweise 1,2-Dichlorethan, bei Temperaturen von 0 bis 150°C umsetzt (Vergleiche auch die Herstellungsbeispiele).

Die zur Durchführung des erfindungsgemäßen Verfahrens d) zur Herstellung der Säurechloride der Formel (V) als Ausgangsstoffe benötigten Säuren sind durch die Formel (VII) allgemein definiert. In dieser Formel (VII) haben Ar², G und Z vorzugsweise bzw. insbesondere diejenigen Bedeutungen, die bereits im Zusammenhang mit der Beschreibung der erfindungsgemäßen Verbindungen der Formel (IVa) als bevorzugt bzw. als insbesondere bevorzugt für Ar², G und Z angegeben wurden.

Die Säuren der Formel (VII) sind teilweise bekannt und/oder können nach bekannten Methoden hergestellt werden (vergleiche z. B. EP-A 178826).

Neu und auch Gegenstand der vorliegenden Anmeldung sind die Säuren der Formel (VIIa), 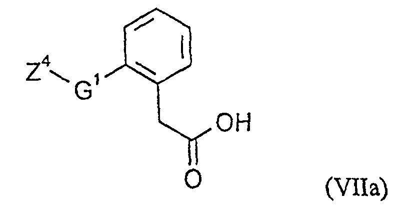 in welcher
- G¹: für -O-CH₂- oder -T-Ar¹-O- steht,
worin T und Ar¹ die oben angegebenen Bedeutungen haben und
- Z⁴: für gegebenenfalls substituiertes Phenyl steht,
wobei die Verbindung 2-(2-Chlorphenoxymethyl)-phenylessigsäure ausgenommen ist.

Die Säuren der Formel (VIIa) werden erhalten (Verfahren e)), wenn man Nitrile der Formel (VIII) 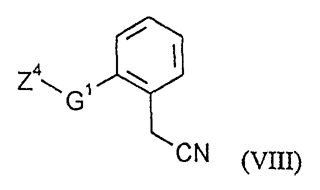 in welcher
- G¹ und Z⁴: die oben angegebenen Bedeutungen haben,
gegebenenfalls in Gegenwart eines Verdünnungsmittels, wie beispielsweise Ethan-1,2-diol, mit einem Alkalimetallhydroxid, wie beispielsweise Natrium- oder Kaliumhydroxid, bei Temperaturen von 0 bis 200°C, vorzugsweise 25 bis 150°C, hydrolysiert.

Die neuen Säuren sind durch die Formel (VIIa) allgemein definiert. In dieser Formel (VIIa) steht G¹ für -O-CH₂- oder -T-Ar¹-O-, worin T und Ar¹ vorzugsweise bzw. insbesondere diejenigen Bedeutungen haben, die bereits im Zusammenhang mit der Beschreibung der erfindungsgemäßen Verbindungen der Formel (I) als bevorzugt bzw. als insbesondere bevorzugt für T und Ar¹ angegeben wurden. Z⁴ steht für gegebenenfalls substituiertes Phenyl, vorzugsweise für gegebenenfalls einfach oder mehrfach, gleich oder verschieden durch Halogen, Cyano, Nitro, Amino, Hydroxy, Formyl, Carboxy, Carbamoyl, Thiocarbamoyl; jeweils geradkettiges oder verzweigtes Alkyl, Alkoxy, Alkylthio, Alkylsulfinyl oder Alkylsulfonyl mit jeweils 1 bis 6 Kohlenstoffatomen; jeweils geradkettiges oder verzweigtes Alkenyl oder Alkenyloxy mit jeweils 2 bis 6 Kohlenstoffatomen; jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen; jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen; jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen; jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen; Cycloalkyl mit 3 bis 6 Kohlenstoffatomen; Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff. Sauerstoff und/oder Schwefel- oder eine Gruppierung 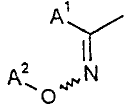 substituiertes Phenyl, worin A¹ und A² vorzugsweise bzw. insbesondere diejenigen Bedeutungen haben, die bereits im Zusammenhang mit der Beschreibung der erfindungsgemäßen Verbindungen der Formel (I) als bevorzugt bzw. als insbesondere bevorzugt für A¹ und A² angegeben wurden. Z⁴ steht insbesondere für gegebenenfalls einfach bis dreifach, gleich oder verschieden durch Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy oder eine Gruppierung 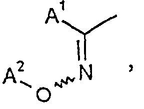 substituiertes Phenyl, worin A¹ und A² vorzugsweise bzw. insbesondere diejenigen Bedeutungen haben, die bereits im Zusammenhang mit der Beschreibung der erfindungsgemäßen Verbindungen der Formel (I) als bevorzugt bzw. als insbesondere bevorzugt für A¹ und A² angegeben wurden.

Die zur Durchführung des erfindungsgemäßen Verfahrens e) zur Herstellung der Säuren der Formel (VIIa) als Ausgangsstoffe benötigten Nitrile sind durch die Formel (VIII) allgemein definiert. In dieser Formel (VIII) haben G¹ und Z⁴ vorzugsweise bzw. insbesondere diejenigen Bedeutungen, die bereits im Zusammenhang mit der Beschreibung der erfindungsgemäßen Verbindungen der Formel (VIIa) als bevorzugt bzw. als insbesondere bevorzugt für G¹ und Z⁴ angegeben wurden.

Die Nitrile der Formel (VIII) sind bekannt und/oder können nach bekannten Methoden hergestellt werden (vergleiche z. B. EP-A 596692).

Die zur Durchführung des erfindungsgemäßen Verfahrens e) weiterhin als Ausgangsstoffe benötigten Alkalimetallhydroxide sind allgemein bekannte Synthesechemikalien.

Die zur Durchführung des erfindungsgemäßen Verfahrens d) weiterhin als Ausgangsstoffe benötigten Halogenierungsmittel sind allgemein bekannte Synthesechemikalien.

Die zur Durchführung des erfindungsgemäßen Verfahrens c) weiterhin als Ausgangsstoffe benötigten Thiole, bzw. Metallthiolate sind durch die Formel (VI) allgemein' definiert. In dieser Formel (VI) hat R¹ vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R¹ angegeben wurde. M steht für Wasserstoff oder ein Metalläqivalent, vorzugsweise für Wasserstoff, Natrium oder Kalium.

Die Thiole, bzw. Metallthiolate der Formel (VI) sind allgemein bekannte Synthesechemikalien.

Die zur Durchführung des erfindungsgemäßen Verfahrens b) weiterhin als Ausgangsstoffe benötigten Ameisensäurederivate sind allgemein bekannte Synthesechemikalien.

Die zur Durchführung des erfindungsgemäßen Verfahrens a) weiterhin als Ausgangsstoffe benötigten Halogenverbindungen sind durch die Formel (III) allgemein definiert. In der Formel (III) hat R² vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R² angegeben wurde. X¹ steht für Halogen, vorzugsweise für Chlor oder Brom.

Die Halogenverbindungen der allgemeinen Formel (III) sind bekannte Reagenzien in der organischen Chemie.

Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im allgemeinen arbeitet man bei Temperaturen von -20°C bis 150°C, vorzugsweise bei Temperaturen von 0°C bis 80°C.

Zur Durchführung des erfindungsgemäßen Verfahrens a) zur Herstellung der Verbindungen der Formel (I) setzt man pro Mol des Hydroxyacrylsäurethiolester der Formel (II) im allgemeinen 1 bis 15 Mol, vorzugsweise 1 bis 8 Mol der Halogenverbindung der Formel (III) ein.

Die erfindungsgemäßen Verfahren a), b) und c) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Getreidekrankheiten, wie beispielsweise Erysiphe-Arten, oder von Krankheiten im Wein-, Obst- und Gemüseanbau, wie beispielsweise Phytophthora- oder Venturia-Arten, oder auch von Reiskrankheiten, wie beispielsweise Pyricularia-Arten, eingesetzt. Mit gutem Erfolg werden auch weitere Getreidekrankheiten, wie Septoria-, Cochliobolus- und Pyrenophora-Arten, bekämpft. Außerdem zeigen die erfindungsgemäßen Wirkstoffe eine besonders starke und breite in vitro Wirkung.

Die Wirkstoffe werden in Abhängigkeit von ihren jeweiligen physikalischen und/oder chemischen Eigenschaften in die üblichen Formulierungen übergeführt, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsülfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

In vielen Fällen werden dabei synergistische Wirkungen beobachtet.

Besonders günstige Mischpartner sind z.B. die folgenden Verbindungen:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazole-5-carboxanilid; 2,6-Dichloro-N-(4-trifluoromethylbenzyl)benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)pyrimidin-4-yloxy]-phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl,.Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenofos, Profenophos,
Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 /5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe werden als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verschäumen, Bestreichen usw.. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel (1)

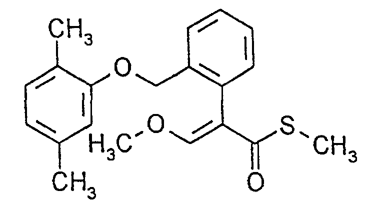

2 g (0,00665 Mol) 2-(2,5-Dimethylphenoxymethyl)-phenylthioessigsäure-S-methylester werden mit 2g (0,0115 Mol) Bis-(dimethylamino)-tert.-butoxymethan 1 Stunde auf 60°C erwärmt. Dann versetzt man mit 6 ml Dimethylformamid und 3 ml konz. wäßriger Salzsäure und erwärmt eine weitere Stunde auf 60°C. Die Reaktionsmischung wird mit Wasser versetzt und mit Dichlormethan extrahiert. Die vereinigten organischen Phasen werden über Natriumsulfat getrocknet und bei vermindertem Druck eingeengt.

Das so erhaltene rohe Zwischenprodukt, 2-[2-(2,5-Dimethylphenoxymethyl)-phenyl]-2-hydroxymethyliden-thioessigsäure-S-methylester, wird in 10 ml Dimethylformamid gelöst, zuerst mit 1,6 g (0,0116 Mol) Kaliumcarbonat und anschließend mit 0,8 g (0,00634 Mol) Dimethylsulfat versetzt und 24 Stunden bei 20°C gerührt. Man gießt auf Wasser, extrahiert mit Dichlormethan, trocknet die organische Phase über Natriumsulfat, destilliert das Lösungsmittel bei vermindertem Druck ab und chromatographiert den Rückstand mit Diethylether/Petrolether (1:1) an Kieselgel. Man erhält 0,5 g (22,9 % der Theorie) 2-[2-(2,5-Dimethylphenoxymethyl)-phenyl]-2-methoxymethyliden-thioessigsäure-S-methylester.

¹H-NMR-Spektrum (CDCl₃/TMS): δ = 2,252 (3H); 2,268 (3H); 3,842 (3H); 4,985 (2H); 6,632/6,651/6,676 (2H); 7,011/7,036 (1H); 7,221-7,254 (1H); 7,318-7,452 (2H); 7,562 (1H); 7,630/7,656 (1H) ppm.

### Herstellung der Ausgangsverbindung:

### Beispiel (IVa-1)

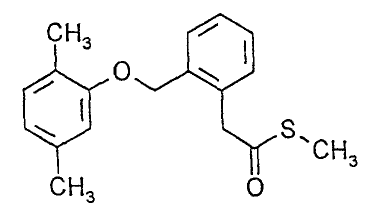

Eine Lösung von 6 g (0,022 Mol) 2-(2,5-Dimethylphenoxymethyl)-phenylessigsäure und 6,6 g (0,055 Mol) Thionylchlorid in 22 ml Dichlorethan wird 4 Stunden auf 60°C erwärmt. Man engt bei vermindertem Druck ein und löst das rohe 2-(2,5-Dimethylphenoxymethyl)-phenylessigsäurechlorid in 50 ml Tetrahydrofuran. Die Lösung wird auf -40°C gekühlt, man gibt unter kräftigem Rühren 1,6 g (0,027 Mol) Natriumthiomethylat zu, und rührt 4 Stunden ohne weitere Kühlung weiter. Man läßt über Nacht stehen, versetzt mit Wasser und extrahiert das Produkt mit Methyl-t-butylether. Man wäscht die organische Phase mit Natriumhydrogencarbonat-Lösung, trocknet über Natriumsulfat, engt bei vermindertem Druck ein und chromatographiert den Rückstand mit Diethylether/Petrolether (1:1) an Kieselgel. Man erhält 2,1 g (31,8 % der Theorie) 2-(2,5-Dimethylphenoxymethyl)-phenylthioessigsäure-S-methylester.

¹H-NMR-Spektrum (CDCl₃/TMS): δ = 2,187 (3H); 2,265 (3H); 2,327 (3H); 3,976 (2H); 5,074 (2H); 6,690/6,715/6,748 (2H); 7,017/7,042 (1H); 7,24-7,364 (3H); 7,507-7,523 (1H) ppm.

### Herstellung des Vorproduktes:

### Beispiel (VIIa-1)

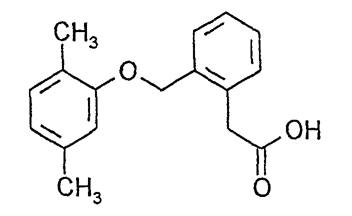

12,6 g (0,05 Mol) 2-(2,5-Dimethylphenoxymethyl)-phenylessigsäurenitril (EP-A 596692, Beispiel 27) werden mit 5,6 g (0,084 Mol) 85 %igem, pulverisiertem Kaliumhydroxid in 50 ml Ethylenglykol 1 Stunde bei 120°C gerührt. Man destilliert das Ethylenglykol im Hochvakuum ab, verteilt den Rückstand zwischen Essigsäureethylester und Wasser, trennt die wäßrige Phase ab und säuert sie mit verdünnter Salzsäure an. Man extrahiert das Produkt mit Dichlormethan, trocknet die organische Phase über Natriumsulfat, destilliert das Lösungsmittel bei vermindertem Druck ab und erhält 6,2 g (45,9 % der Theorie) 2-(2,5-Dimethylphenoxymethyl)phenylessigsäure, die ohne weitere Reinigung umgesetzt wird.

¹H-NMR-Spektrum (CDCl₃/TMS): δ = 2,163 (3H); 2,312 (3H); 3,723 (2H); 5,062 (2H); 6,684/6,709/6,738 (2H); 7,0/7,025 (1H); 7,3-7,4 (4H); 7,452-7,494 (1H) ppm.

### Beispiel 2:

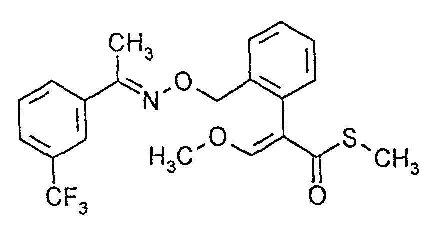

Eine Mischung von 2,5 g (0,0066 Mol) {2-[1-(3-Trifluormethylphenyl)-ethylidenaminooxymethyl]-phenyl}-thioessigsäure-S-methylester und 1,26 g (0,0072 Mol) Bis-(dimethylamino)-tert.-butoxymethan wird eine Stunde auf 60°C erwärmt. Dann versetzt man mit 20 ml Dimethylformamid und 5 ml 2 normaler wäßriger Salzsäure und erwärmt eine weitere Stunde auf 60°C. Hierauf gibt man Wasser zu, extrahiert das Produkt mit Essigsäureethylester, trocknet über Natriumsulfat getrocknet und engt bei vermindertem Druck ein. Das rohe Zwischenprodukt, 2-Hydroxymethyliden-2-{2-[1-(3-trifluormethylphenyl)-ethyliden-aminooxymethyl]-phenyl}-thioessigsäure-S-methylester, löst man in 6 ml Dimethylformamid und rührt das Gemisch nach Zugabe von 1,69 g (0,012 Mol) Kaliumcarbonat und 0,77 g (0,0061 Mol) Dimethylsulfat 24 Stunden bei Raumtemperatur. Die Mischung wird auf Wasser gegossen, mit Dichlormethan extrahiert. Die organische Phase wird abgetrennt, über Natriumsulfat getrocknet und bei vermindertem Druck eingeengt. Der Rückstand wird mit Diethylether/Petrolether (1:1) an Kieselgel chromatographiert. Man erhält 0,6 g (21,5 % der Theorie) 2-Methoxymethyliden-2-{2-[1-(3-trifluormethylphenyl)ethyliden-aminooxymethyl]-phenyl}-thioessigsäure-S-methylester.

¹H-NMR-Spektrum (CDCl₃/TMS): δ = 2,245 (3H); 2,259 (3H); 3,863 (3H); 5,306 (2H); 7,2-8,0 (9H) ppm.

### Herstellung:

### Beispiel (IVa-2)

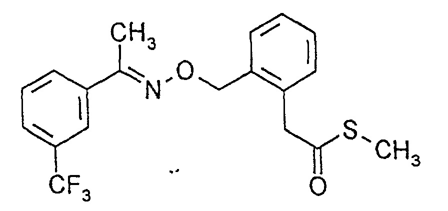

Eine Lösung von 3,5 g (0,01 Mol) {2-[1-(3-Trifluormethylphenyl)-ethylidenaminooxymethyl]-phenyl}-essigsäure und 1,4 g (0,012 Mol) Thionylchlorid in 10 ml Dichlorethan wird 4 Stunden unter Rückfluß erhitzt. Man engt bei vermindertem Druck ein und löst das rohe {2-[1-(3-Trifluormethylphenyl)-ethylidenaminooxymethyl]-phenyl}-essigsäurechlorid in 20 ml Tetrahydrofuran. Die Lösung wird auf -40°C gekühlt, man gibt unter kräftigem Rühren 0,7 g (0,01 Mol) Natriumthiomethylat zu, und rührt 4 Stunden ohne weitere Kühlung weiter. Man läßt über Nacht stehen, versetzt mit Wasser und extrahiert das Produkt mit tert.-Butylmethylether. Man wäscht die organische Phase mit Natriumhydrogencarbonat-Lösung, trocknet über Natriumsulfat, engt bei vermindertem Druck ein und chromatographiert den Rückstand mit Diethylether/Petrolether (1:1) an Kieselgel. Man erhält 2,5 g (65,6 % der Theorie) {2-[1-(3-Trifluormethylphenyl)-ethylidenaminooxymethyl]-phenyl}-thioessigsäure-S-methylester.

¹H-NMR-Spektrum (CDCl₃/TMS): δ = 2,236 (3H); 2,272 (3H); 4,027 (2H); 5,305 (2H); 7,259-7,894 (8H) ppm.

### Herstellung des Vorproduktes:

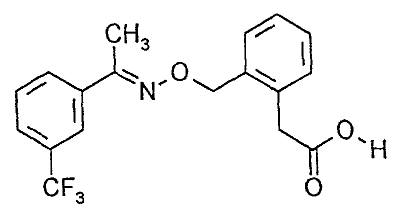

15 g (0,045 Mol) {2-[1-(3-Trifluormethylphenyl)-ethylidenaminooxymethyl]-phenyl}essigsäurenitril werden in 90 ml Ethylenglykol mit 7 g (0,106 Mol) 85 %-igem KOH-Pulver 7 Stunden bei 140°C gerührt. Man gießt auf Wasser, extrahiert mit Essigsäureethylester. Dann säuert man an und extrahiert das Produkt mit Dichlormethan. Man erhält nach dem Abziehen des Lösungsmittels 13 g (82,2 % der Theorie) {2-[1-(3-Trifluormethylphenyl)-ethylidenaminooxymethyl]-phenyl}essigsäure.

¹H-NMR-Spektrum (CDCl₃/TMS):
δ = 2,181 (3H); 3,826 (2H); 5,291 (2H); 7,248-7,339 (3H); 7,421-7,468 (2H); 7,570/7,596 (1H); 7,761/7,787 (1H); 7,858 (1H) ppm.

### Anwendungsbeispiele:

### Beispiel A

### Phytophthora-Test (Tomate) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von *Phytophthora infestans* inokuliert

Die Pflanzen werden in einer Inkubationskabine mit 100% relativer Luftfeuchtigkeit und ca. 20°C aufgestellt.

3 Tage nach der Inokulation erfolgt die Auswertung.

Bei diesem Test zeigen z.B. die folgenden Verbindungen (1) und (2) der Herstellungsbeispiele bei einer Wirkstoffkonzentration von 100 ppm einen Wirkungsgrad von 77 %.

### Beispiel B

### Venturia-Test (Apfel) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden bespritzt man junge Pflanzen mit der Wirkstoffzubereitung taufeucht besprüht.. Nach Antrocknen des Spritzbelages werden die Pflanzen mit einer wäßrigen Konidiensuspension des Apfelschorferregers *Venturia inaegualis* inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Bei diesem Test zeigen z.B. die folgenden Verbindungen der Herstellungsbeispiele (1) und (2) bei einer Wirkstoffkonzentration von 10 ppm einen Wirkungsgrad von 100 %.

### Beispiel C

### Erysiphe-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von *Erysiphe gramiris f.sp.hordei* bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei diesem Test zeigt z.B. die folgende Verbindung (2) bei einer Wirkstoffaufwandmenge von 250 g/ha einen Wirkungsgrad von 100 %.

### Beispiel D

### Pyricularia-Test (Reis) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit werden man junge Reispflanzen mit der Wirkstoffzubereitung taufeucht besprüht. 4 Tage nach dem Abtrocknen des Spritzbelages werden die Pflanzen mit einer wäßrigen Sporensuspension von *Pyricularia oryzae* inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Bei diesem Test zeigen z.B. die folgenden Verbindungen der Herstellungsbeispiele (1) und (2) bei einer Wirkstoffkonzentration von 0,05 % einen Wirkungsgrad von 80 bis 100%.

## Patentansprüche

1. Verbindungen der Formel (I) gemäß Anspruch 1 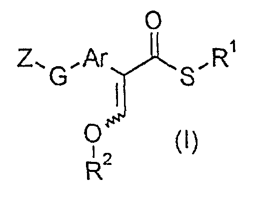 in welcher
Ar für ortho-Phenylen steht,
G für -O-CH₂- oder 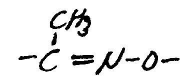 steht,
R¹ für Methyl steht,
R² für Methyl steht, und
Z für jeweils gegebenenfalls einfach bis dreifach, gleich oder verschieden durch Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy, substituiertes Phenyl, steht.

2. Schädlingsbekämpfungsmittel, **gekennzeichnet durch** einen Gehalt an mindestens einer Verbindung der Formel (I) nach Anspruch 1.

3. Verfahren zur Bekämpfung von Schädlingen, **dadurch gekennzeichnet, daß** man Verbindungen der Formel (I) nach Anspruch 1 auf Schädlinge und/oder ihren Lebensraum einwirken läßt.

4. Verwendung von Verbindungen der Formel (I) nach Anspruch 1 zur Bekämpfung von Schädlingen.

5. Verfahren zur Herstellung von Schädlingsbekämpfungsmitteln, **dadurch gekennzeichnet, daß** man Verbindungen der Formel (I) nach Anspruch 1 mit Streckmitteln und/oder oberflächenaktiven Mitteln vermischt.

6. Verfahren zur Herstellung von Verbindungen der Formel (I) nach Anspruch 1, **dadurch gekennzeichnet, daß** man Hydroxyacrylsäurethiolester der allgemeinen Formel (II) 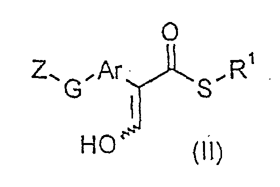 in welcher
Ar, G, R¹ und Z die in Anspruch 1 angegebenen Bedeutungen haben,
mit einer Halogenverbindung der allgemeinen Formel (III)
X¹-R² (III)
in welcher
X¹ für Halogen steht und
R² die in Anspruch 1 angegebene Bedeutung hat,
gegebenenfalls in Gegenwart eines Säureakzeptors und gegebenenfalls in Gegenwart eines Verdünnungsmittels umsetzt.

7. Verbindungen der Formel (II) 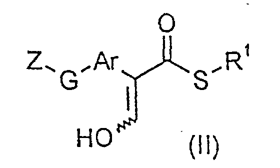 in welcher R¹, Ar und Z die in Anspruch 1 angegebenen Bedeutungen haben, und G für 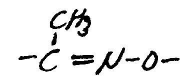 steht.

## Claims

1. Compounds of the formula (I) 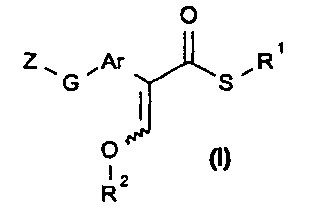 in which
Ar represents ortho-phenylene,
G represents -O-CH₂- or 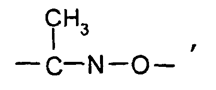
R¹ represents methyl,
R² represents methyl, and
Z represents phenyl, which is optionally mono- to trisubstituted by identical or different substituents selected from the group consisting of methyl, ethyl, n- or i-propyl, n-, i-, s- or t-butyl, trifluoromethyl, methoxy, ethoxy, n- or i-propoxy, difluoromethoxy, difluorochloromethoxy, trifluoroethoxy and trifluoromethoxy.

2. Pesticides, **characterized in that** they comprise at least one compound of the formula (I) according to Claim 1.

3. Method for controlling pests, **characterized in that** compounds of the formula (I) according to Claim 1 are allowed to act on pests and/or their habitat.

4. Use of compounds of the formula (I) according to Claim 1 for controlling pests.

5. Process for preparing pesticides, **characterized in that** compounds of the formula (I) according to Claim 1 are mixed with extenders and/or surfactants.

6. Process for preparing compounds of the formula (I) according to Claim 1, **characterized in that** hydroxyacrylic thiol esters of the general formula (II) 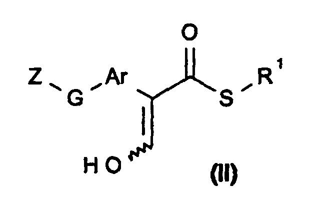 in which
Ar, G, R¹ and Z are each as defined in Claim 1,
are reacted with a halogen compound of the general formula (III)
X¹-R² (III)
in which
X¹ represents halogen and
R² is as defined in Claim 1,
if appropriate in the presence of an acid acceptor and
if appropriate in the presence of a diluent.

7. Compounds of the formula (II) 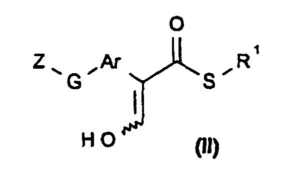 in which R¹, Ar and Z are each as defined in Claim 1 and G represents

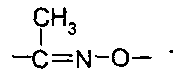

## Revendications

1. Composés de formule (I) selon la revendication 1, 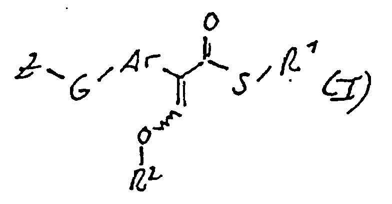 dans laquelle
Ar est un groupe orthophénylène
G est un groupe -O-CH₂- ou 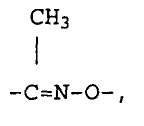
R¹ est un groupe méthyle,
R² est un groupe méthyle et
Z est un groupe phényle portant le cas échéant 1 à 3 substituants, identiques ou différents, méthyle, éthyle, n-propyle, iso-propyle, n-butyle, iso-butyle, sec.-butyle, tertio-butyle, trifluorométhyle, méthoxy, éthoxy, n-propoxy, iso-propoxy, difluorométhoxy, difluorochlorométhoxy, trifluoréthoxy, trifluorométhoxy.

2. Compositions pesticides, **caractérisées par** une teneur en au moins un composé de formule (I) suivant la revendication 1.

3. Procédé pour combattre des parasites, **caractérisé en ce qu'**on fait agir des composés de formule (I) suivant la revendication 1 sur les parasites et/ou sur leur milieu.

4. Utilisation de composés de formule (I) suivant la revendication 1 pour combattre des parasites.

5. Procédé de préparation de compositions pesticides, **caractérisé en ce qu'**on mélange des composés de formule (I) suivant la revendication 1 avec des diluants et/ou des agents tensio-actifs.

6. Procédé de production de composés de formule (I) suivant la revendication 1, **caractérisé en ce qu'**on fait réagir des thioesters d'acides hydroxyacryliques de formule générale (II) 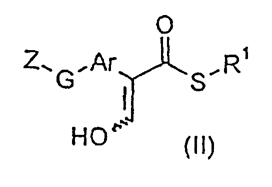 dans laquelle
Ar, G, R¹ et Z ont les définitions indiquées dans la revendication 1,
avec un composé halogéné de formule générale (III)
X¹-R² (III)
dans laquelle
X¹ représente un halogène et
R² a la définition indiquée dans la revendication 1,
le cas échéant en présence d'un accepteur d'acide et en présence éventuelle d'un diluant.

7. Composés de formule (II) 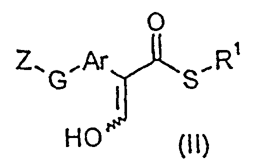 dans laquelle R¹, Ar et Z ont les définitions indiquées dans la revendication 1 et G est un groupe
